# EUROPEAN PATENT SPECIFICATION

(11) **EP 0 917 467 B1**
(45) Date of publication and mention of the grant of the patent: **19.02.2003**
(21) Application number: 97930452.4
(22) Date of filing: 01.07.1997
(51) Int. Cl.: A61K 35/20, A61P 1/00, A61P 29/00

(54) **PHARMACEUTICAL FORMULATIONS CONTAINING COLOSTRUM AND THEIR USE**
KOLOSTRUM ENTHALTENDE PHARMAZEUTISCHE ZUSAMMENSETZUNGEN UND DEREN VERWENDUNG
FORMULATIONS PHARMACEUTIQUES CONTENANT DU COLOSTRUM, ET LEUR UTILISATION

(30) Priority: 02.07.1996 IT MI961354; 31.10.1996 IT MI962275
(43) Date of publication of application: 26.05.1999
(73) Proprietor: Pharmaproducts UK Limited, Liverpool, Merseyside L2 9TL (GB)
(72) Inventor: FERRARIO, Gianluigi, Road Town, Tortola (VG); BARTORELLI, Luisa, I-20135 Milano (IT)
(74) Representative: Bianchetti, Giuseppe, Prof.
(86) International application number: EP9703411
(87) International publication number: WO98000149

(56) References cited:
- EP-A- 0 527 283
- WO-A-93/25227
- WO-A-96/34614

## Description

The present invention relates to the use of colostrum for the treatment of intestine inflammatory diseases and anorectal disturbances.

The etiology of chronic inflammatory diseases of the intestine, such as Crohn' disease, ulcerative colitis and segmentary ileitis, is still unknown: a number of immunological, bacterial, dietetic and psychosomatic factors have been considered, but up to now no definite hypothesis have been proved. The treatment of said diseases involves, in addition to dietetic measures, the use of corticosteroids, immunosuppressors, antibiotics, antibacterials and psychoactive drugs. Since such diseases have a chronic course, or relapses and remissions alternate, the need for a treatment alternative to the long-term ones with the above mentioned drugs is evident. Finally, in the most serious cases, which do not respond to any treatment, surgery is taken into consideration.

Anorectal disturbances such as hemorrhoids, anorectal fistulas and anal fissures can be the outcome of vascular problems; of inflammatory conditions of the intestinal tract, or they can be of a traumatic or congenital origin. All of said conditions are characterized by a remarkable worsening of the symptoms (pain, bleeding) following defecation, therefore the treatment consists in the use of rectum lubricants and of agents making stools softer (natural or semi-synthetic polysaccharides and cellulose derivatives such as bran, psyllium and methylcellulose) thanks to an increased absorption of liquids in the intestinal tract. However, such an approach is not always sufficient to resolve the condition, and it is often necessary to resort to surgery, which often involves a long, rather painful post-surgery course. Therefore a valid alternative to surgery is highly desirable.

The present invention aims at overcoming the above cited problems, by the use of pharmaceutical formulations containing equine, bovine or anyhow animal, but preferably equine, colostrum, for the treatment of intestine inflammatory diseases and anorectal disturbances.

The present invention further relates to the use of equine, bovine or anyhow animal, but preferably equine, colostrum, for the treatment of intestine inflammatory diseases and anorectal disturbances.

Colostrum has been already proposed as an active ingredient for the therapy of hepatic diseases (EP-A-652013) and of multiple sclerosis (EP-A-46909). Topical dermatological compositions of colostrum are known from WO-A-9416675.

It has surprisingly been found that colostrum, administered to patients suffering from intestine inflammatory diseases and anorectal disturbances, exerts a remarkable antiinflammatory and epithelizing action on the involved tract of intestine, thus improving the general conditions of the treated patient, particularly causing the disappearance of bleeding, abdomen cramps and diarrhoea crisis typical of these conditions. Moreover, colostrum acts favourably inducing effectively and physiologically the evacuation, without causing any side-effects.

In fact, clinical trials on 20 patients, showed a favourable response of 17 patients to the treatment with colostrum, attaining in very short times a remarkable improvement in the symptoms.

The pharmaceutical formulations of invention contain powder or liquid colostrum in amounts of 500 mg to 5 g per unitary dose.

More particularly, the formulations of the invention comprise gastro-resistant formulations such as tablets containing powdered, freeze-dried or sprayzed colostrum or pearls containing liquid colostrum, as well as rectal formulations such as suppositories containing powdered, freeze-dried or sprayzed colostrum or enemas containing liquid colostrum.

The formulations of invention are prepared according to the conventional pharmaceutical techniques for the preparation of gastro-resistant and rectal formulations.

The enteric coating is used to protect colostrum against the gastric acid environment, such a coating can consist of copolymerized methacrylic acid/methacrylic acid methyl esters, such as those known under the names Eudragit^{R} (commercialized by Rohm Pharma), polymers such as cellulose acetate phthalate, hydroxypropyl methylcellulose phthalate, water-based polymer dispersions, such as Eudragit^{R} (by Rohm Pharma), Aquateric^{R} (FMC Corp.). The enteric coating can optionally contain plasticizers such as triacetin, cetanol, citric and phthalic acids esters.

The formulations of the invention can optionally contain other active ingredients known to have a beneficial effect on the condition to treat.

According to a further aspect of the invention, the formulations can contain colostrum mixed with milk.

The following examples further illustrate the invention.

### Example 1

| One enteric-coated tablet contains: | |
|---|---|
| Powder colostrum | 110 mg |
| Hydroxypropyl methylcellulose phthalate | 6 mg |
| Cetyl alcohol | 0.5 mg |

The enteric coating solution is applied on the colostrum core by fluidized bed coating.

### Example 2

| One enteric-coated tablet contains: | |
|---|---|
| Powder colostrum | 64 mg |
| Powder milk | 45 mg |
| Hydroxypropyl methylcellulose phthalate | 32 mg |
| Cetyl alcohol | 5 mg |

The enteric coating solution is applied on the colostrum core by fluidized bed coating.

### Example 3

| One suppository contains: | |
|---|---|
| Powder colostrum | 250 mg |
| Saturated polyglycosylated glycerides | 150 mg |
| Solid semi-synthetic glycerides | 2600 mg |

### Example 4

| Enema | |
|---|---|
| Powder colostrum | 300 mg |
| Saturated polyglycosylated glycerides | 150 mg |
| Saturated triglycerides | 1550 mg |

## Claims

1. The use of colostrum for the preparation of a medicament for the treatment of intestine inflammatory diseases and anorectal disturbances.

## Patentansprüche

1. Verwendung von Kolostrum zur Herstellung eines Arzneimittels zur Behandlung von entzündlichen Darmkrankeiten und anorektalen Störungen.

## Revendications

1. Utilisation du colostrum pour la préparation d'un medicament utile destiné au traitement des maladies inflammatoires intestinales et des affections ano-rectales.
